# EUROPEAN PATENT APPLICATION

(11) **EP 4 803 637 A1**
(43) Date of publication of application: **09.09.2026**
(21) Application number: 25382212.6
(22) Date of filing: 07.03.2025
(51) Int. Cl.: C12Q 1/6886

(54) **METHOD FOR MONITORING OF CANCER USING MINIMAL RESIDUAL DISEASE ANALYSIS**

(71) Applicant: Fundación Incliva, 46010 Valencia (ES); Universitat de Valéncia, 46010 Valencia (ES)
(72) Inventor: MARTÍN ARANA, Jorge, 46010 Valencia (ES); TARAZONA LLAVERO, Noelia, 46010 Valencia (ES); CERVANTES RUIPÉREZ, Andrés, 46010 Valencia (ES); GIMENO VALIENTE, Francisco, 46010 Valencia (ES)
(74) Representative: Cueto, Sénida

(57) **Abstract**

The present invention discloses a method for detecting minimal residual disease (MRD) in a subject comprising performing whole exome sequencing of cell-free DNA from a plasma sample obtained before surgery selection the most relevant differentially expressed variants, and detecting said variants in a plasma sample obtained at a later time point.

## Description

### Technical Field

Provided herein is a method to monitor cancer using minimal residual disease analysis. The method comprises whole exome sequencing from plasma samples, and processing the variations identified to determine the presence of a cancer.

### Background

Post-surgery (cell tumor DNA) ctDNA assessment is crucial for guiding adjuvant treatment (ACT) decisions. While existing assays detect ctDNA and help identify high-risk patients for recurrence, their limited sensitivity post-surgery poses challenges in deciding on ACT. Additionally, a significant portion of patients fail to clear ctDNA post ACT, leading to recurrence. To improve patient management, there is a need to enhance the sensitivity of ctDNA detection assays and deepen our understanding of colorectal cancer progression mechanisms.

EP4473132A1; EP3833783B1; EP3134541B1 and EP3430170B1 disclose methods for monitoring cancer using minimal residual disease analysis. However, they disclose a customized panel of biomarkers to detect, not an unbiased whole exome sequencing (WES), neither a method to identify the most relevant alterations.

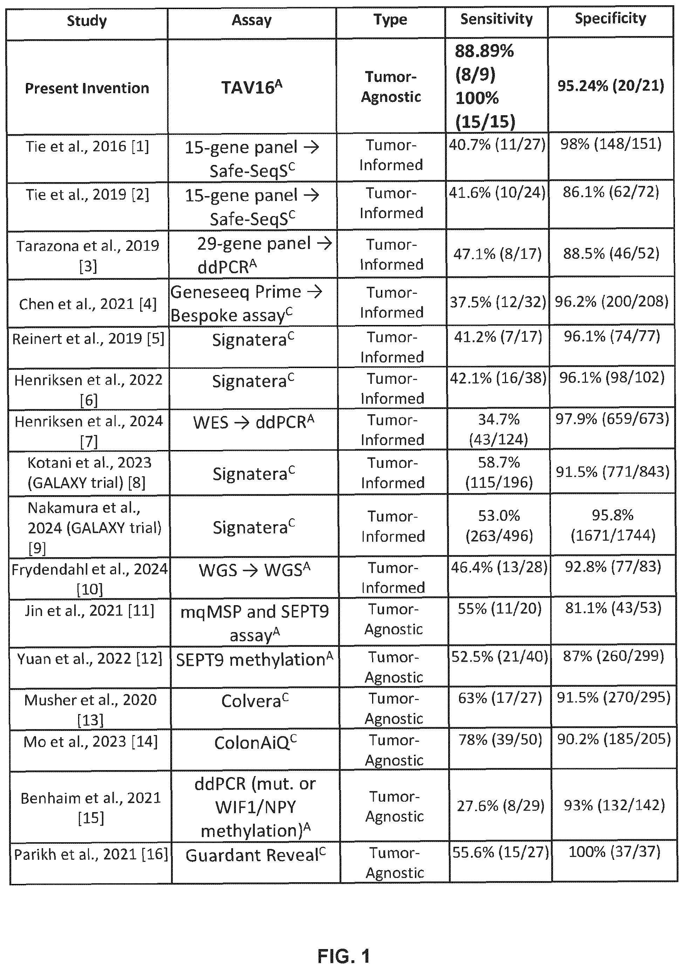

The present invention discloses a method that involves WES of ctDNA at baseline, followed by tracking the most frequently mutated variants at different time points to predict relapse in patients across two independent cohorts. This approach demonstrates higher sensitivity in detecting minimal residual disease (MRD) compared to current assays. The method of the invention has an improved sensitivity compared to any other method of the state of the art (Fig. 1)

The method of the invention reduces uncertainty and provides peace of mind to the patients. With greater accuracy than current methods, it helps doctors make timely decisions, whether intensifying treatment for high-risk individuals or avoiding unnecessary chemotherapy. This minimizes toxicity, side effects, and psychological burden, allowing survivors to regain confidence in their lives.

In conclusion, the present invention is a breakthrough innovation that enhances cancer management through a simple, tumor-agnostic blood test for real-time recurrence detection, eliminating invasive biopsies. The method further improves patient's life quality and eases the economic burden of healthcare systems.

### Description

The term **"nucleotide"** is intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the term "nucleotide" includes those moieties that contain hapten or fluorescent labels and may contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The term **"nucleic acid"** and **"polynucleotide"** are used interchangeably herein to describe a polymer of any length, e.g., greater than about 2 bases, greater than about 10 bases, greater than about 100 bases, greater than about 500 bases, greater than 1000 bases, greater than 10,000 bases, greater than 100,000 bases, greater than about 1,000,000, up to about 1010 or more bases composed of nucleotides, e.g., deoxyribonucleotides or ribonucleotides, and may be produced enzymatically or synthetically, which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine, thymine, uracil (G, C, A, T and U respectively). DNA and RNA have a deoxyribose and ribose sugar backbone, respectively.

As used herein, the term **"variation"** or **"variant"** may correspond to genetic aberration of a genetic locus, particularly an exon. The genetic aberration may a tumor associated alteration. The genetic aberration may be a copy number alterations (CNAs), copy number losses (CNLs), single nucleotide variants (SNVs), insertions or deletions (indels). For example, a sequence variation can be referred to by the position of the variation and which type of substitution (e.g., G to A, G to T, G to C, A to G, etc. or insertion/deletion of a G, A, T or C, etc.) is present at the position. A sequence variation may be a substitution, deletion, insertion or rearrangement of one or more nucleotides. In the context of the present method, a sequence variation can be generated by, e.g., a PCR error, an error in sequencing or a genetic variation. It can refer to a variation (e.g., a nucleotide substitution, an indel or a rearrangement) that is present or deemed as being likely to be present in a nucleic acid sample. A genetic variation can be from any source. For example, a variation or variant can be generated by a mutation (e.g., a somatic mutation), an organ transplant or pregnancy. In many cases, the term terms "variation" or "variant" can be replaced by the term "mutation". For example, if the method is being used to detect variants that are associated with cancer or other diseases that are caused by mutations, then "variation" or "variant" can be replaced by the term "mutation".

As used herein, the term **"calling"** means indicating or detecting whether a particular sequence variant is present in a sample. This may involve, for example, providing a sequence that contains the sequence variation and/or annotating a sequence having the sequence variation, indicating that the sequence has an A to T variation at a specific position.

The term **"plasma sample,"** as used herein, denotes a plasma sample containing nucleic acids, that can be obtained from peripheral blood. Nucleic acid samples used herein may be complex in that they contain multiple different molecules that contain sequences. Genomic DNA samples from a mammal (e.g., mouse or human) are types of complex samples. Complex samples may have more than about 10⁴, 10⁵ , 10⁶ or 10⁷, 10⁸, l0⁹ or 10¹⁰ different nucleic acid molecules.

As used herein, the terms **"cell-free DNA from the bloodstream"** "circulating cell-free DNA" and cell-free DNA" ("cfDNA") refers to DNA that is circulating in the peripheral blood of a patient. The DNA molecules in cell-free DNA may have a median size that is below 1 kb (e.g., in the range of 50 bp to 500 bp, 80 bp to 400 bp, or 100-1,000bp), although fragments having a median size outside of this range may be present. Cell-free DNA may contain circulating tumor DNA (ctDNA), i.e., tumor DNA circulating freely in the blood of a cancer patient or circulating fetal DNA (if the subject is a pregnant female). cfDNA can be obtained by centrifuging whole blood to remove all cells, and then isolating the DNA from the remaining plasma or serum. Such methods are well known (see, e.g., Lo et al, Am J Hum Genet 1998; 62:768-75). Circulating cell-free DNA can be double-stranded or single-stranded. This term is intended to encompass free DNA molecules that are circulating in the bloodstream as well as DNA molecules that are present in extra-cellular vesicles (such as exosomes) that are circulating in the bloodstream.

As used herein, the term **"circulating tumor DNA"** (or "ctDNA") is tumor-derived DNA that is circulating in the peripheral blood of a patient. ctDNA is of tumor origin and originates directly from the tumor or from circulating tumor cells (CTCs), which are viable, intact tumor cells that shed from primary tumors and enter the bloodstream or lymphatic system. The precise mechanism of ctDNA release is unclear, although it is postulated to involve apoptosis and necrosis from dying cells, or active release from viable tumor cells. ctDNA can be highly fragmented and in some cases can have a mean fragment size about 100-250 bp, e.g., 150 to 200 bp long. The amount of ctDNA in a sample of circulating cell-free DNA isolated from a cancer patient varies greatly: typical samples contain less than 10% ctDNA, although many samples have less than 1% ctDNA and some samples have over 10% ctDNA. Molecules of ctDNA can be often identified because they contain tumorigenic mutations.

As it will be shown later, the present invention discloses a ctDNA analysis from 40 patients with localized CC who underwent curative-intent surgery but subsequently experienced recurrence. The study, applied WES at different timepoints. The objective was to surpass the constraints of custom panels by investigating whether a plasma-based WES approach could improve the sensitivity to detect MRD Zviran, A., Schulman, R.C., Shah, M. et al. Genome-wide cell-free DNA mutational integration enables ultra-sensitive cancer monitoring. Nat Med 26, 1114-1124 (2020). Additionally, we sought to uncover mechanisms underlying the progression of localized colorectal cancer that could reveal potential therapeutic approaches for effectively eliminating MRD.

The first object of the invention relates to an *in vitro* method for detecting minimal residual disease (MRD) in a subject suffering or that has suffered cancer, comprising the following steps:
(a) assaying cell free deoxynucleic acids cfDNA from a plasma sample obtained from said subject at a first time point; the first-time point being pre-surgery or immediately after surgery, preferably pre-surgery
(b) detecting altered variants from said cfDNA from a plasma sample; by using whole exome sequencing;
   using genomic DNA from white blood cells (WBC) from the same subject as germline control to asses said altered variants
(c) computer processing the altered variants to determine the most relevant altered variants, and
(d) generating an amplicon panel design that comprises sequences of at least two, or 3, or 5, or 5, or 6, or 7 or 8 or 9 or 10 or 11 or 12, preferably at least the 13 most relevant altered variants;

assaying
   cfDNA from a plasma sample from the same subject obtained at a second time point, which is after surgery; and
cfDNA from plasma samples from a pool of healthy subjects as a control;
   with said amplicon panel, to call the presence of least one of said most relevant altered variants,
wherein the calling of at least one of said most relevant altered variants in the plasma sample at the second time point indicates the presence of minimal residual disease (MRD) in said subject.

The method of the invention comprises a computer analysis of the data generated by WES of cfDNA from a sample obtained from a subject.

The method of the invention with WES of cfDNA immediately after surgery (WES-TA approach), significantly improves the sensitivity for detecting MRD. In two independent localized colorectal cancer cohorts, one comprising 25 relapsed and 21 non-relapsed patients (discovery cohort), and the other comprising 15 relapsed patients (validation cohort), Sensitivity reached 86.7% and 100% in the discovery and validation cohorts, respectively, with a specificity of 95%, surpassing previous studies utilizing personalized assays based on a tumor-informed or tumor-agnostic approach with custom panels.

The method of the invention is also termed TAV16, preferably the first-time point is immediately after surgery, its advantages are improved cost-effectiveness. The TAV16 approach yielded sensitivity values similar to those obtained from a WES approach when considering one mutation for ctDNA positivity. This finding, combined with the observation that the concordance between plasmas at different time points is stronger than that observed between primary tissue and plasma, demonstrates that leveraging a personalized tumor-agnostic assay based on plasma WES at diagnosis, rather than relying on primary tumor, is be pivotal in developing a robust approach for monitoring MRD.

The method of the invention involves isolating fragments of cell free DNA from two or more plasma samples, where the first biological sample is obtained at a first time point and a second or subsequent biological sample is obtained at a later time point; assaying cfDNA means isolating fragments of cfDNA from a plasma sample, constructing two or more cell-free DNA libraries each containing cell-free DNA fragments from the plasma samples, wherein the libraries are each prepared by direct ligation of adapters; sequencing the libraries to at least about 50X, preferably at least about 100X, more preferably at least about 150X, even more preferably at least about 200X, even more preferably at least about 250X, even more preferably at least about 300X, or at least 350X exome-wide sequencing coverage at baseline; and at least 20000x, preferably at least 50000x, more preferably at least 80000x, even more preferably at least 100000x in the targeted sequencing, to detect at least one of the most relevant altered variations in the plasma sample at a second time point for quality control to both the cfDNA from plasma samples and the plasma sample obtained from the pool of healthy subjects, or panel of normals (PON), wherein the calling of at least one common variant between the first time point and the second time point indicates MRD; the first time point is pre-surgery, simultaneous to surgery or immediately after surgery; the second or subsequent time point is after the first time point

In a particular embodiment the first time point can be pre-surgery or simultaneously to the surgery or immediately after surgery, preferably pre-surgery. Pre-surgery means at least 2 days, preferably at least 1 day, more preferably at least 12 hr, even more preferably at least 6 hr, even more preferably at least 2 hr before surgery, or 30 minutes to 1 hour before surgery; simultaneously to the surgery means during the surgery with curative purpose to remove the tumor; immediately after surgery means from 1 minute to 10 minutes after surgery or 1 minute to 30 minutes after surgery; or 1 minute to 1 hour after surgery; or 1 minute to 6 hours after surgery. The surgery is curative-intent surgery.

The variants may correspond to genetic aberration of a genetic locus, particularly an exon. The genetic aberration may a tumor associated alteration. The genetic aberration may be a copy number alterations (CNAs), copy number losses (CNLs), single nucleotide variants (SNVs), insertions or deletions (indels). The variants may be identified in a variety of nucleic acid types. For example, the tumor associated alteration may be identified in cfDNA. The tumor associated alteration may comprise changes in allelic expression, or gene expression. Preferably the method of the invention comprises the detection of nucleotide variants (SNVs) and small insertions and deletions (INDELs). In the present specification "variant detection" and "variant calling" are synonyms.

The variants are detected by whole exome sequencing ("WES") is a technique used to sequence all the expressed genes in a genome (known as the exome). It includes first selecting only the subset of DNA that encodes proteins (exons), and then sequencing the exons using any DNA sequencing technology well known in the art or as described herein. In a human being, there are about 180,000 exons, which constitute about 1% of the human genome, or approximately 30 million base pairs. To sequence the exons of a genome, fragments of double-stranded genomic DNA are obtained (e.g., by methods such as sonication, nuclease digestion, or any other appropriate methods). Linkers or adapters are then attached to the DNA fragments, which are then hybridized to a library of polynucleotides designed to capture only the exons. The hybridized DNA fragments are then selectively isolated and subjected to sequencing using any sequencing method known in the art or described herein.

In one embodiment, the sequencing of a DNA fragment is carried out using commercially available sequencing technology SBS (sequencing by synthesis) by Illumina. In another embodiment, the sequencing of the DNA fragment is carried out using chain termination method of DNA sequencing. In yet another embodiment, the sequencing of the DNA fragment is carried out using one of the commercially available next-generation sequencing technologies, including SMRT sequencing from Pacific Biosciences, Ion Torrent^{™} sequencing from ThermoFisher Scientific, Pyrosequencing from Roche, and SOLiD^{®} technology from Applied Biosystems. Any appropriate sequencing technology may be chosen for sequencing.

As used herein, the term "coverage" refers to the percentage of the genome covered by reads. Coverage also refers to, in shotgun sequencing, the average number of reads representing a given nucleotide in the reconstructed sequence. It can be calculated from the length of the original genome (G), the number of reads(N), and the average read length(L) as N × L /G. Biases in sample preparation, sequencing, and genomic alignment and assembly can result in regions of the genome that lack coverage (that is, gaps) and in regions with much higher coverage than theoretically expected. It is important to assess the uniformity of coverage, and thus data quality, by calculating the variance in sequencing depth across the genome. The term depth may also be used to describe how much of the complexity in a sequencing library has been sampled. All sequencing libraries contain finite pools of distinct DNA fragments. In a sequencing experiment only some of these fragments are sampled. In an embodiment, the whole exome sequencing coverage is at least about 50X, preferably at least about 100X, more preferably at least about 150X, even more preferably at least about 200X, even more preferably at least about 250X, even more preferably at least about 300X, or at least 350X exome-wide sequencing coverage and at least 80x preferably at least 100x in the gDNA from WBC sample.

The advantages of the computer processing and the plasma monitoring is the possibility to detect the top variant allele frequency that will not been detected through a tumor-informed assay. WES provides a broader scope for detecting pathogenic mutations compared to tumor-informed approaches This lack of detection reduces the possibility of effectively eliminating MRD and excludes these patients from the opportunity to receive experimental treatments within clinical trials. Moreover, enrolling patients in clinical trials remains a significant challenge, largely due to prolonged screening periods, the need for sequential tissue biopsies, and time-consuming genotyping processes. The method of the invention provides solutions to these problems.

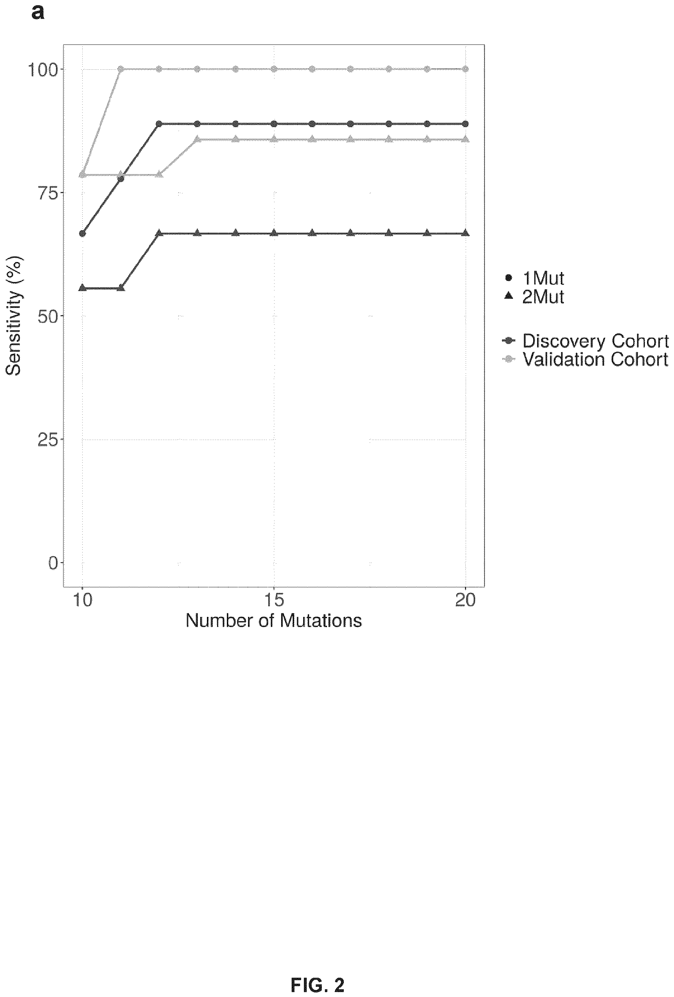

In a particular embodiment the most relevant altered variants are at least 2 variants, preferably at least 3 variants, more preferably at least 4 variants, even more preferably at least 5 variants, even more preferably at least 10 variants, even more preferably at least 13 variants, even more preferably at least 15 variants; even more preferably at least 16 variants. At least 13 variants are preferred so the method of the invention achieves the highest sensitivity values (Fig. 2a). However, a skilled person would understand that appropriate sensitivity values can be obtained with less variants, such as 14, 13, 12, 11 and 10.

In a preferred embodiment, the top 16 variants with the highest variant allele frequency (VAF) in the plasma baseline are determined, these are the 16 most relevant variants. As it will be shown later, sensitivity did not improve with the selection of more than 16 candidate alterations. Thus, a maximum of 16 candidates or variants is preferred. Particularly, the variants are somatic mutations. They are identified as follows

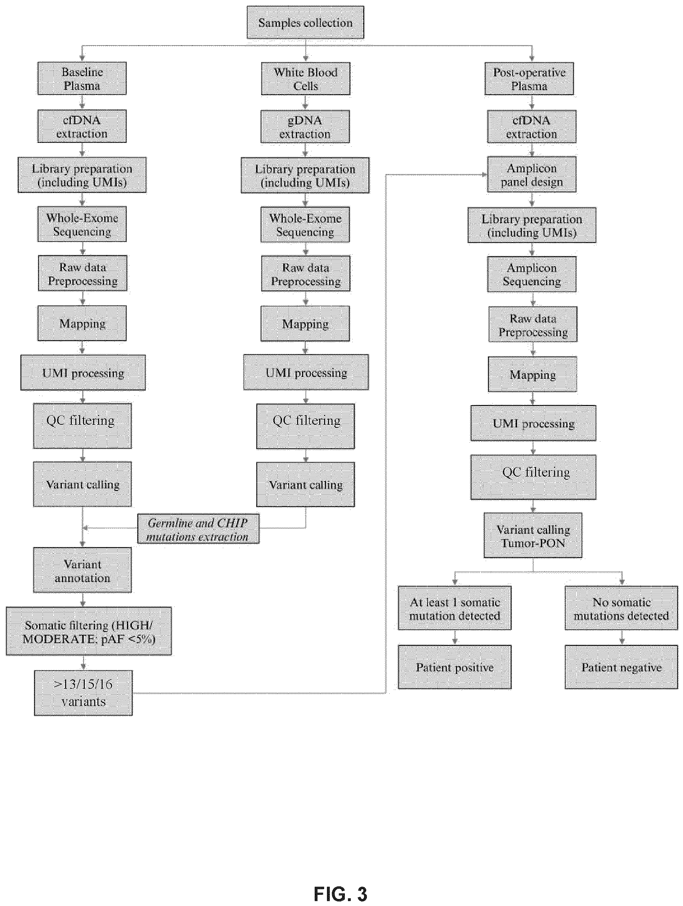

From a collected plasma sample, the method of the invention comprises, the extraction of cfDNA (cell-free DNA) from plasma and gDNA (genomic DNA) from mononuclear cells, this is WBC (Fig. 3). Preferably, WBC are obtained from a plasma sample obtained at the same first time point that the one of the cfDNA is analyzed. However, a SKP would understand the WBC can be obtained from a plasma sample obtained at different time points, as WBC serves as genomic germline control and this is invariable during the whole life of the subject.

In the case of plasma cfDNA, a minimum requirement of more than 40 ng of DNA is preferably established to ensure the quality of the analysis. Library preparation involves the fragmentation and ligation of tumor DNA, gDNA, and cfDNA using unique dual-index and Unique Molecular Identifiers (UMI) adapters. The process follows standard protocols with modifications, including the incorporation of UMI adapters during ligation and a pre-capture PCR step with specific primer sets. For cfDNA, adapter concentration is adjusted based on input DNA, the number of pre-capture PCR cycles is optimized by each particular sample using routine work and experimentation, and an additional bead-based purification step is introduced before ethanol clean-up.

Once the libraries are ready, the whole exome sequencing of both cfDNA and gDNA is performed on a high-throughput platform with paired-end reads, including an extended read for UMI detection. and generating raw data to be used in the analysis. Computer analysis can be carried out in parallel for data from plasma sample and WBC. This process comprises several steps, all of them performed following standard procedures in the field:
1. Quality Control: Raw sequencing reads undergo quality assessment to evaluate overall sequence quality, GC content, and adapter contamination.
2. Adapter Trimming and Quality Filtering: Adapters are removed and reads are filtered out with a mean quality score below Q30 to retain high-confidence sequences.
3. Read Alignment: Processed reads are mapped to the hg38 human reference genome, ensuring accurate sequence alignment.
4. Binary Alignment Map (BAM) Post-Processing: The resulting BAM files undergo processing following GATK best practices, which include duplicate marking, base quality score recalibration, and alignment refinement. Samples with a median coverage of less than 350x or less than 300x or less than 250x or less than 200x or less than 150x or less than 100x. or less than 50x are discarded.
5. UMI Processing: UMIs are extracted and the reads originating from the same UMI family are deduplicated to eliminate PCR artifacts and improve variant calling accuracy.
6. Variant Calling: Mutations in plasma, and normal samples are identified using at least one variant calling softwares to enhance sensitivity and specificity. In particular, variants detected by at least one of the variant callers used are considered for downstream variant filtering. For example, 3 different software can be used and if a variant is detected by at least one of them it is considered for the next step.
7. Germline Variant Detection: A parallel pipeline to identify germline variants is processed, ensuring proper distinction from somatic mutations, this is, altered variants in the same subject. This pipeline consists of a variant calling step using specific software to detect germline mutations.
8. Variant Filtering:
   ∘ A minimum VAF threshold of 0.1% preferably 0.01% is applied on plasma samples (cfDNA) and 5% preferably, 10% on normal samples (gDNA).
   ∘ The final variant set is obtained by intersecting the outputs of both variant callers.
   ∘ Variants detected in normal samples (gDNA) are filtered out.
   ∘ Variants identified as sequencing artifacts based on a blacklist are removed.
   ∘ Clonal hematopoiesis of indeterminate potential (CHIP) variants are excluded from plasma samples to prevent false positives.
9. Variant Annotation: The curated variant list is annotated to determine functional impact. Somatic pathogenic variants are further classified using public mutation database, and previously sequenced cfDNA from subjects that can uncover novel pathogenic variants.
10. Prioritization and candidate mutations selection: High and moderate impact somatic mutations are selected. Only mutations with a population frequency of less than 5% are considered. From the resulting somatic mutational profile, the 13, preferably 15 or even more preferably 16 variants with the highest variant allele frequency that meet the criteria are selected as candidate mutations for monitoring. These are the most relevant altered variants.

The classification of variants into High, moderate and low impact is done according to the state of the art. The most commonly used classification follows variant effect predictors (VEPs) like SnpEff, Ensembl VEP, or ClinVar annotations.

High-impact variants (SNVs) are expected to have a severe effect on protein function, often resulting in loss of function (LoF) or gain-of-function (GoF) in key oncogenes or tumor suppressors, for example nonsense mutations (such as the introduction of a premature stop codon); frameshift mutations (such as the disruption of protein translation); splice site mutations (such as the ones that affect normal mRNA splicing).

Moderate-impact variants (SNVs) alter protein function, but the effect is less drastic than high-impact variants. They may reduce protein activity compared to the wild type, for example missense mutations, in-frame insertions/deletions.

Low-impact variants (SNVs) are generally neutral or have minimal impact on protein function, for example synonymous mutations, that do not change the amino acid sequence (e.g., silent mutations).

Once the most relevant altered variants are determined, a plasma sample from the same subject obtained at a second time point is examined for the presence of at least one of those most relevant altered variants.

A skilled person would understand that the determination of the most relevant altered variants does not necessarily have to be done before the plasma sample of the second time point is obtained. For example, the plasma sample of the first and second time points can be obtained, frozen or conserved by any other method until the WES assay, and then examine the presence of at least one of those most relevant altered variants obtained in the WES assay of the plasma sample of the first time point in the plasma sample of the second time point.

In a particular embodiment, the second time point is post-surgery and after the first-time point. In a particular embodiment, the second time point is at least 1, at least 2, at least 3, at least 4, at least 5, or at least 6 weeks after the first time point, preferably between 3 and 4 weeks after surgery. Preferably the time point is at least 3 weeks after the surgery, as false negatives reduce from that date. During the first 3 weeks after surgery, strong inflammation leads to an increased release of non-tumoral cfDNA, which may dilute or obscure the detection of ctDNA containing one or more of the most relevant altered variants. Subsequent time points are also possible. The surgery is curative-intent surgery

After cfDNA extraction from the plasma sample obtained at the second time point, a customized amplicon panel is designed for each patient, this is, plurality of probe nucleic acids. This panel includes a specific set of amplicons and their respective primer pairs, designed to cover each of the 16 variants of the previous step.

Subsequently, library preparation and targeted sequencing of the plasma sample is performed. In a particular embodiment, complementary sequencing of a control sample (wild-type) is performed, which consists of a mixture of DNA extracted from peripheral blood of a set of healthy subjects, forming the so-called "panel of normals" (PON). A new PON can be obtained for each analysis or reuse. A panel of normals may comprise a group of 2, 3, 4, 5, 10, 15, 20, 25, or 30 healthy subjects. The same PON can be used for one or more subjects. The sample derived from the combination plasma samples from healthy subjects can be reused but taking into account that the most relevant altered variants will be different from a subject to another, so a different amplicon panel needs to be designed and analyzed in each case.

MRD detection often requires detecting trace amounts of ctDNA (<0.1%) through ultra-deep sequencing. As the mutation allele frequency proportions of ctDNA in MRD monitoring samples are often below the sequencing and PCR error rate, differentiating true mutations from sequencing and PCR errors is required. The use of Unique Molecular Identifiers (UMIs) can enable identifying reads from the same single strand molecules and suppressing sequencing and late-stage PCR errors during the consensus fragment building process. A single-stranded UMI is used for NGS assays. UMIs are used in combination with the Amplicon Panel to enhance the detection of the most relevant altered variants previously identified. **"Amplicon Panel"** refers to a predefined set of DNA target regions selected for amplification and sequencing, designed to detect specific altered variants.

Variant calling in the plasma sample obtained at a second time point is performed as follows, steps 1 to 4 are performed identically to steps 1 to 4 of the WES described above:
1. Quality Control: Raw sequencing reads undergo quality assessment to evaluate sequence quality, GC content, and adapter contamination.
2. Adapter Trimming and Quality Filtering: Adapters are removed, and reads are filtered out with a mean quality score below Q30 to retain high-confidence sequences.
3. Read Alignment: Processed reads are mapped to the hg38 human reference genome, ensuring accurate sequence alignment.
4. BAM Post-Processing: The resulting BAM files undergo post-processing following GATK best practices, including duplicate marking, base quality score recalibration, and alignment refinement.
5. UMI Processing and Error Correction: UMIs are extracted and reads originating from the same UMI family are deduplicated. Single-molecule UMI processing is optimized for amplicon-based sequencing data to ensure highly accurate molecular tracking. A minimum depth coverage threshold of at least 20,000x, preferably at least 50,000, even more preferably at least 100,000× is applied in quality control for both plasma samples and the PON to ensure sufficient sequencing depth for error correction.
6. Variant Calling with Background Error Correction: Paired variant calling was performed from this using a heuristic method and a statistical test, based on the VarScan2 software, using the number of aligned reads supporting each allele. In particular, variant calling in plasma is performed using a paired-sample approach that incorporates a second time-point plasma sample along with PON analysis, providing additional background error correction beyond UMI-based filtering. To significantly enhance the accuracy of variant detection, the following considerations are taken into account:
   ∘ A minimum VAF threshold of 0.01%, or minimum 0.1% is applied on plasma samples.
   ∘ Variants detected in PON samples are filtered out.
   ∘ Only those mutations detected exclusively in the plasma samples and classified as "SOMATIC" are considered as true somatic variants.
7. MRD detection: A patient was considered positive if at least one somatic mutation of the at least 13, 15 or 16 most relevant altered variants was detected through this analysis.

In the present specification "Minimal Residual Disease" and "Molecular Relapse" are synonyms. Another object of the invention relates to an *in vitro* method for detecting molecular relapse in a subject suffering or that has suffered cancer, comprising the following steps: a) to d) of the preceding method to detect MRD, but additionally subsequent plasma samples at different time points after surgery are obtained, wherein the calling of at least one variant, of the most relevant altered variants in one of the subsequent plasma samples of those variants identified in the plasma sample from the first time point indicates a molecular relapse of said subject.

In a particular embodiment, the subsequent plasma samples are obtained at least 1, 2, 3 or 4 weeks after the preceding one. For example, one plasma sample per month for a period of 6 or 12 months after surgery. The analysis of said subsequent samples is performed identically to step d) of the method to detect MRD.

The advantage of this method is that it will provide valuable information to the clinician to determine whether the adjuvant treatment provided to the patient after the surgery is effective or it is necessary to change the treatment. This is determined by the presence or absence of the at least one of the most relevant alterations determined from the plasma sample of the first time point.

In a particular embodiment, the cancer is selected from the group consisting of: genitourinary cancer, breast cancer, lung cancer, prostate cancer, colorectal cancer, melanoma, bladder cancer, non-Hodgkin lymphoma, kidney cancer, endometrial cancer, leukemia, pancreatic cancer, thyroid cancer, and liver cancer, and any combination thereof. In a preferred embodiment, the cancer is colorectal cancer. In some embodiments, the subject is asymptomatic for said cancer.

In the present specification, "subject" and "patient" are synonyms and is meant a mammal, including, but not limited to, a human or non-human mammal, such as a bovine, equine, canine, ovine, rodent, or feline.

A "reference genome" is a defined genome used as a basis for genome comparison or for alignment of sequencing reads thereto. A reference genome may be a subset of or the entirety of a specified genome; for example, a subset of a genome sequence, such as exome sequence, or the complete genome sequence.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

Unless specifically stated or obvious from context, as used herein, the term "or" is understood to be inclusive. Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the invention are set forth in examples below

### Brief description of the drawings

**Figure 1****.** Sensitivity and specificity of the main ctDNA published studies for detecting MRD. Sensitivity and specificity are calculated based on the total number of recurring and non-recurring patients, respectively. C, Commercial assay; A, Academic assay; ddPCR, droplet digital PCR; WES, whole-exome sequencing; WGS, whole-genome sequencing.
   [1] Tie J, Wang Y, Tomasetti C, et al. Circulating tumor DNA analysis detects minimal residual disease and predicts recurrence in patients with stage II colon cancer. Sci Transl Med 2016; 8: 346ra92.
   [2] Tie J, Cohen JD, Wang Y, et al. Circulating Tumor DNA Analyses as Markers of Recurrence Risk and Benefit of Adjuvant Therapy for Stage III Colon Cancer. JAMA Oncol 2019; 5: 1710-1717.
   [3] Tarazona N, Gimeno-Valiente F, Gambardella V, et al. Targeted next-generation sequencing of circulating-tumor DNA for tracking minimal residual disease in localized colon cancer. Ann Oncol 2019; 30: 1804-1812.
   [4] Chen G, Peng J, Xiao Q, et al. Postoperative circulating tumor DNA as markers of recurrence risk in stages II to III colorectal cancer. J Hematol OncolJ Hematol Oncol 2021; 14: 80.
   [5] Reinert T, Henriksen TV, Christensen E, et al. Analysis of Plasma Cell-Free DNA by Ultradeep Sequencing in Patients With Stages I to III Colorectal Cancer. JAMA Oncol 2019; 5: 1124-1131.
   [6] Henriksen TV, Tarazona N, Frydendahl A, et al. Circulating Tumor DNA in Stage III Colorectal Cancer, beyond Minimal Residual Disease Detection, toward Assessment of Adjuvant Therapy Efficacy and Clinical Behavior of Recurrences. Clin Cancer Res 2022; 28: 507-517.
   [7] Henriksen TV, Demuth C, Frydendahl A, et al. Unraveling the potential clinical utility of circulating tumor DNA detection in colorectal cancer-evaluation in a nationwide Danish cohort. Ann Oncol 2024; 35: 229-239.
   [8] Kotani D, Oki E, Nakamura Y, et al. Molecular residual disease and efficacy of adjuvant chemotherapy in patients with colorectal cancer. Nat Med 2023; 29: 127-134.
   [9] Nakamura Y, Tsukada Y, Matsuhashi N, et al. Multiomic analysis for minimal residual disease detection: Addressing challenges in stage II-III colon cancer from COSMOS-CRC-01. J Clin Oncol 2024; 42: 180-180.
   [10] Frydendahl, A., Nors, J., Rasmussen, M. H., Henriksen, T. V., Nesic, M., Reinert, T., ... & Andersen, C. L. (2024). Detection of circulating tumor DNA by tumor-informed whole-genome sequencing enables prediction of recurrence in stage III colorectal cancer patients. European Journal of Cancer, 211, 114314.
   [11] Jin S, Zhu D, Shao F, et al. Efficient detection and post-surgical monitoring of colon cancer with a multi-marker DNA methylation liquid biopsy. Proc Natl Acad Sci 2021; 118: e2017421118.
   [12] Yuan Z, Wang S, Ni K, et al. Circulating Methylated SEPT9 DNA Analyses to Predict Recurrence Risk and Adjuvant Chemotherapy Benefit in Stage II to III Colorectal Cancer. Med Sci Monit; 28. Epub ahead of print 2 September 2022. DOI: 10.12659/MSM.937757.
   [13] Musher BL, Melson JE, Amato G, et al. Evaluation of Circulating Tumor DNA for Methylated BCAT1 and IKZF1 to Detect Recurrence of Stage II/Stage III Colorectal Cancer (CRC). Cancer Epidemiol Biomarkers Prev 2020; 29: 2702-2709.
   [14] Mo, S., Ye, L., Wang, D., Han, L., Zhou, S., Wang, H., ... & Cai, G. (2023). Early detection of molecular residual disease and risk stratification for stage I to III colorectal cancer via circulating tumor DNA methylation. JAMA oncology, 9(6), 770-778.
   [15] Benhaim, L., Bouché, O., Normand, C., Didelot, A., Mulot, C., Le Corre, D., ... & Laurent-Puig, P. (2021). Circulating tumor DNA is a prognostic marker of tumor recurrence in stage II and III colorectal cancer: multicentric, prospective cohort study (ALGECOLS). European Journal of Cancer, 159, 24-33.
   [16] Parikh AR, Van Seventer EE, Siravegna G, et al. Minimal Residual Disease Detection using a Plasma-only Circulating Tumor DNA Assay in Patients with Colorectal Cancer. Clin Cancer Res 2021; 27: 5586-5594.

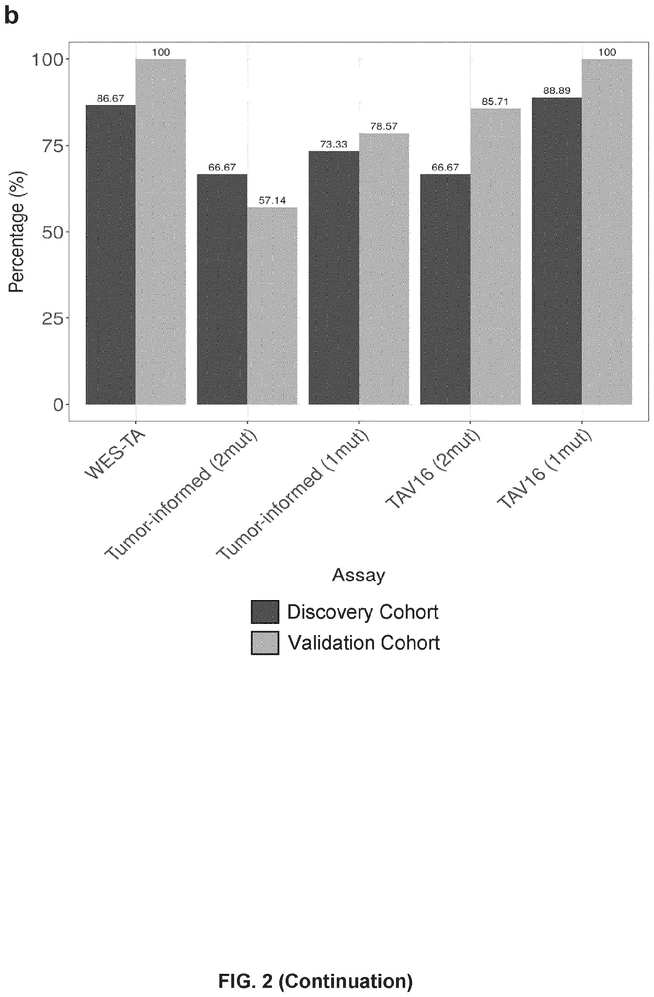

**Figure 2****.** TAV16 design and performance. **a.** Minimal residual disease detection sensitivity based on varying numbers of tracked mutations for the discovery and validation cohorts. Sensitivity values are presented for two criteria: determining a patient as ctDNA-positive by identifying at least one somatic mutation (altered variants) (1Mut) or by detecting two somatic mutations (altered variants) (2Mut). **b,** Sensitivity values achieved from the different assays evaluated in the discovery and validation cohorts,
**Figure 3****.** Schematic diagram of an embodiment of the method of the invention.

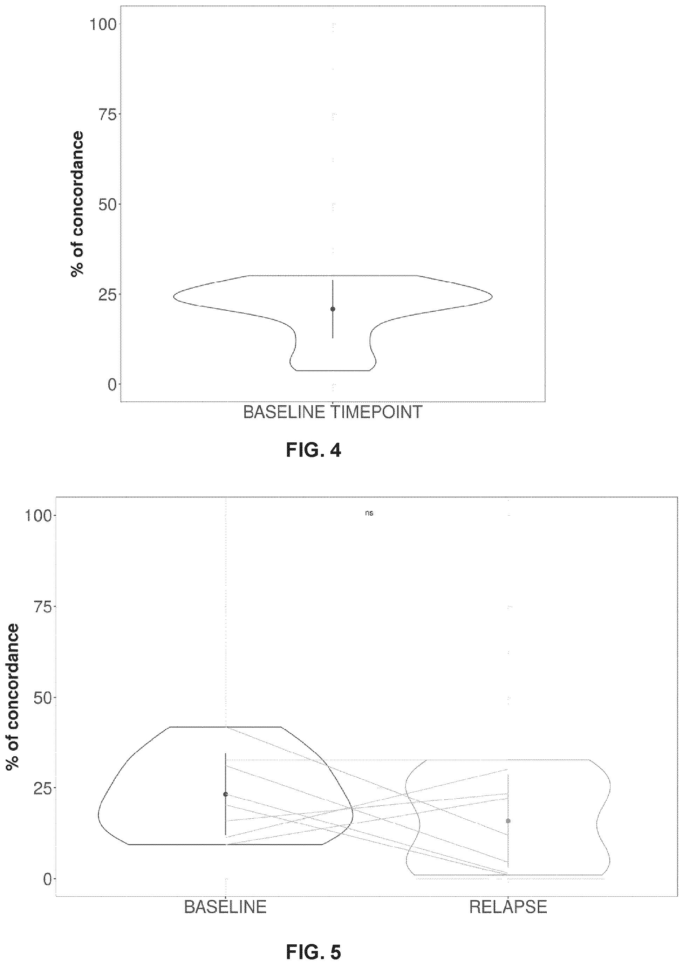

**Figure 4****.** Concordance analysis of primary tumor and plasma baseline somatic SNVs. The cohort's median concordance is represented by a dot.
**Figure 5****.** Percentage of concordance in somatic SNVs between primary tumor and plasma at baseline (n = 12) and at relapse (n = 17) among the patient cohort. Data are presented as median values +/- standard deviation. Concordance is determined by comparing each patient with themselves at different stages.

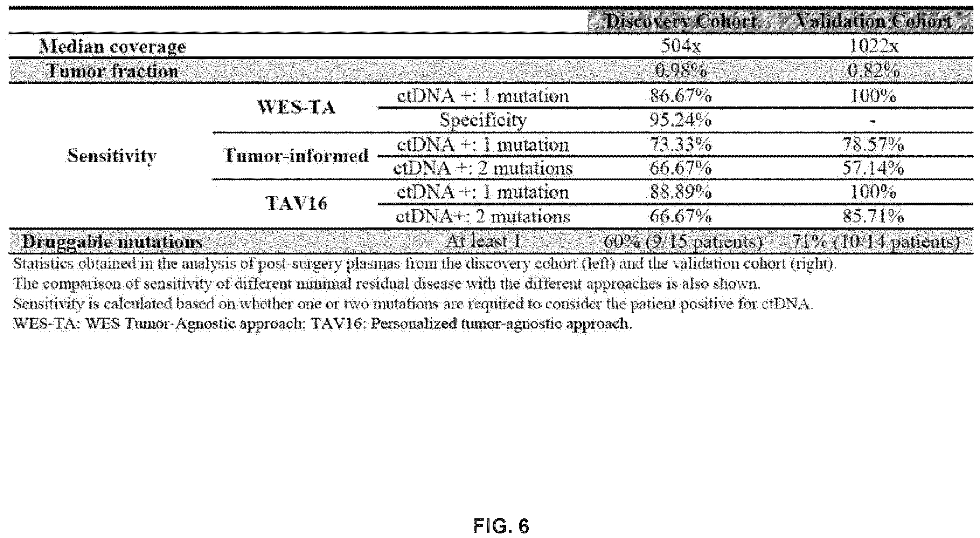

**Figure 6****.** Minimal Residual Disease Detection. Statistics obtained in the analysis of post-surgery plasmas from the discovery cohort (left) and the validation cohort (right). The comparison of sensitivity of different minimal residual disease with the different approaches is also shown. Sensitivity is calculated based on whether one or two mutations are required to consider the patient positive for ctDNA. WES-TA: WES Tumor-Agnostic approach; TAV16: Personalized tumor-agnostic approach; embodiment of the method of the invention.

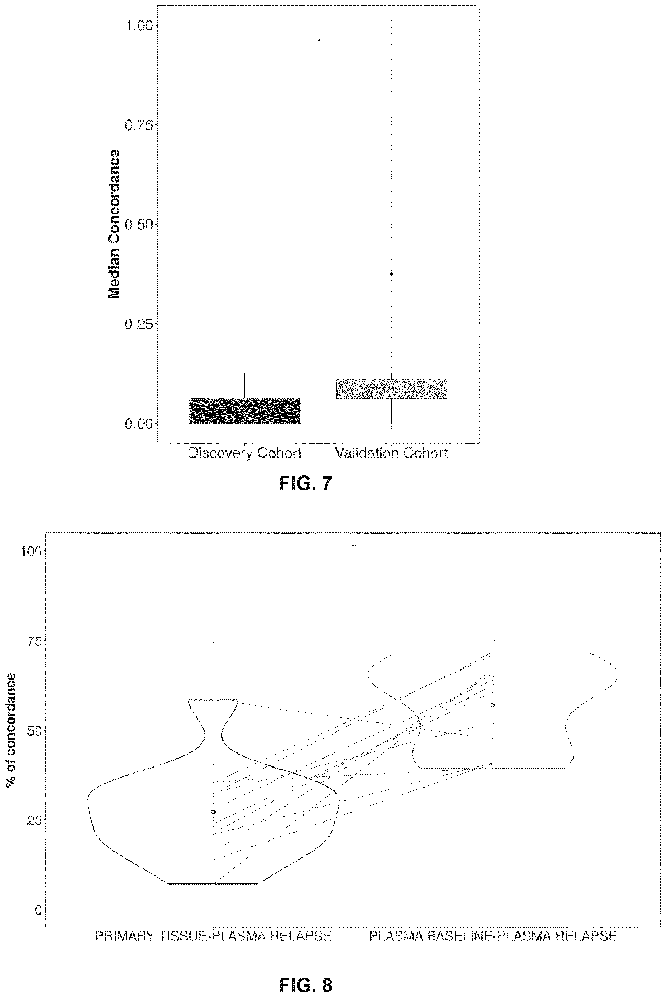

**Figure 7****.** Concordance of candidate variants when selecting the 16 somatic mutations with the highest VAF for MRD monitoring in primary tumor and plasma baseline samples within the discovery cohort (left) and the validation cohort (right).
**Figure 8****.** Concordance comparison between the primary tumor and plasma at relapse (n=25) versus the concordance of plasma at both baseline and relapse (n=12) of somatic mutations across the discovery cohort. Data are presented as median values +/- standard deviation. Concordance is calculated by comparing each patient with themselves at different stages.

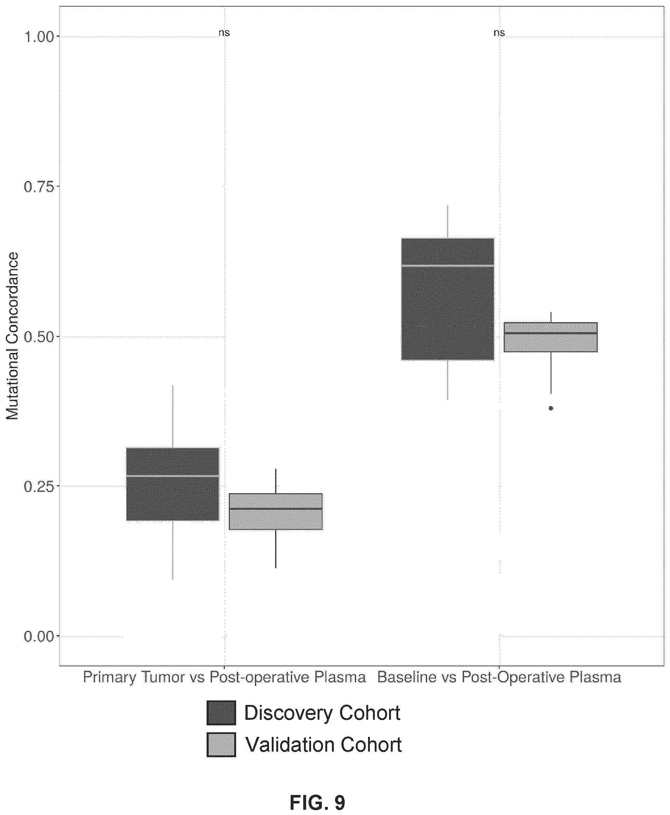

**Figure 9****.** Concordance comparison between the primary tumor and plasma at post-surgery (n=15) versus the concordance of plasma at both baseline and relapse (n=12) of somatic mutations across the discovery cohort. Data are presented as median values +/- standard deviation. Concordance is calculated by comparing each patient with themselves at different stages.

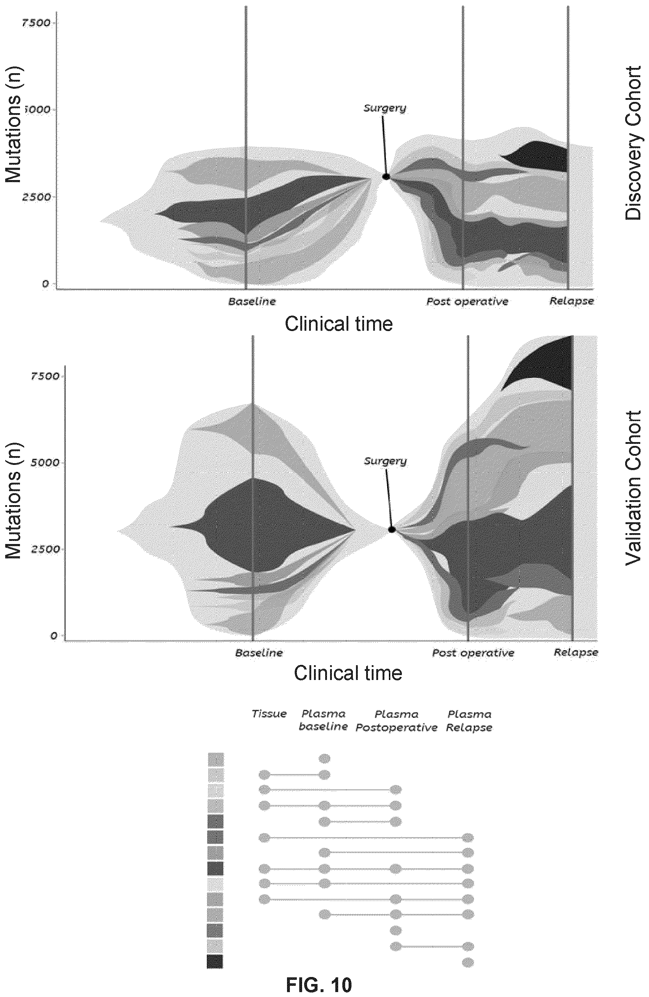

**Figure 10****.** Plasma monitoring relevance. Evolutionary plot in the discovery cohort for 7 paired patients (top) and the validation cohort for 14 paired patients (bottom), illustrating somatic mutations occurring at baseline, post-surgery, and at relapse. Black mutations represent those variants acquired during the evolution process. The indications of the sampling time points are not drawn to time-scale. Moving along the chromatic scale from green to purple signifies mutations persisting over time and considered clonal. Mutations emerging after surgery until relapse are represented in shades of red, indicating clones arising during tumor evolution in this period. The upset plot on the right indicates the correspondence of colors with temporal points where the mutation is found.

### Examples

### Methods

### Patients and study design

Through a liquid biopsy program within the Digestive Tumors Group at the Hospital Clinico Universitario in Valencia (Spain), we prospectively enrolled 320 patients diagnosed with localized CC who had experienced recurrence between July 2015 and May 2021. Use of ACT was at the discretion of the treating clinician. As an inclusion criterion for the study, patients who had experienced relapse and had plasma at relapse and tissue at baseline available were selected. Additionally, 21 patients who had not experienced relapse and had sufficient quantities of plasma post-surgery were also included in the analysis. In total, 25 patients in the discovery cohort were studied. Primary tumor tissue, collected at diagnosis before any treatment, and plasma at relapse, refers to plasma collected from a patient a few days after the detection of disease progression on the CT scan, before starting treatment for advanced disease, were collected from all patients. Of the 25 patients with recurrence, 48% (n=12) had plasma available preoperatively (considered as baseline time point) and 60% (n=15) postoperatively, and matching tissue from the recurrence lesion was also available in 68% (n=17) patients. All patients peripheral blood was collected in EDTA tubes at diagnosis, 6-8 weeks post-surgery, prior ACT, and upon disease progression for ctDNA and WBC analysis. cfDNA was extracted from 4 mL of plasma for each patient. Tumor tissue DNA was extracted at diagnosis and relapse following macrodissection of samples to ensure >70% cellular content for subsequent DNA and RNA extraction. WES was performed on the collected samples. RNA deconvolution from primary tissue and proteomics from both primary (n = 25) and relapse (n = 17) tumor tissue of the discovery cohort were utilized to validate the findings from WES. To validate these results, we used an external cohort consisting of 15 CC patients from seven hospitals in Denmark, in whom primary tissue, plasma at diagnosis, and plasma at relapse were collected. Plasma was isolated within 2 hours of blood collection by double centrifugation at 3005 g for 10 minutes and stored at -80°C until DNA extraction. Postoperative plasma for analysis was available for 14 of the 15 patients (93%). All patients provided written informed consent, and the study was conducted in accordance with the Declaration of Helsinki. Further information on research design is available in the Nature Research Reporting Summary linked to this article.

### DNA extraction

Discovery cohort: Macrodissection of the FFPE block with primary tissue was performed in each patient. Samples from relapses were also employed if available. DNA was extracted using AllPrep DNA/RNA FFPE kit (Qiagen) for tumoral DNA from FFPE cuts, Chemagic DNA blood (Chemagen) for germline DNA from matched white blood cells (WBC) and QIAamp Circulating Nucleic Acid kit (QIAGEN) for cfDNA from 4 mL plasma samples. All extraction protocols were performed according to the corresponding manufacturer's instructions. Tumor DNA and WBC was quantified using QuantiFluor dsDNA System (Promega), whereas cfDNA quality and quantity were assessed with Cell-free DNA ScreenTape Assay (Agilent). cfDNA samples were not accepted if cfDNA content was below 70%.

Validation cohort: DNA was extracted from fresh-frozen tumor tissue using the Puregene DNA purification kit (Gentra Systems) and from FFPE samples with the QiAamp DNA FFPE tissue kit (Qiagen). WBC DNA was extracted from the buffy coat using the Qiasymphony DNA mini kit (Qiagen). DNA from tumor and WBC was quantified by the Qubit^{™} dsDNA BR Assay Kit (ThermoFisher). Cell-free DNA was purified from 4-8 mL of plasma using the QIAamp Circulating Nucleic Acids kit (Qiagen) and quantified with droplet digital PCR (Bio-Rad Laboratories), using assays targeting regions on Chr3 and Chr7, as described previously in Reinert T, Schøler LV, Thomsen R, Tobiasen H, Vang S, Nordentoft I, Lamy P, Kannerup AS, Mortensen FV, Stribolt K, Hamilton-Dutoit S, Nielsen HJ, Laurberg S, Pallisgaard N, Pedersen JS, Ørntoft TF, Andersen CL. Analysis of circulating tumour DNA to monitor disease burden following colorectal cancer surgery. Gut. 2016 Apr;65(4):625-34. doi: 10.1136/gutjnl-2014-308859. Epub 2015 Feb 4. PMID: 25654990.

### Whole exome sequencing

Discovery cohort: Libraries were prepared using 100ng inputs of tumor DNA, 100ng of WBC DNA and 10-40ng of cfDNA. KAPA HyperPlus (Roche) with IDT UDI-UMI indexes (IDT) was used for library preparation of tissue and WBC DNA, according to the instructions of KAPA HyperCap Workflow v3. The only modification applied was the use of 5 mL of 15 mM UDI-UMI at the ligation step, instead of a universal adaptor, and posterior pre-capture PCR with Illumina Primer Mix. KAPA HyperPrep (Roche) with the same adapters was selected for cfDNA, with the following changes to the mentioned protocol: i) the adaptor was diluted proportionally with the input DNA, with a maximum of 12 mM for 40ng; ii) pre-capture PCR was performed with a total of 11 cycles; iii) post-PCR purifications were performed with 50 mL of KAPA HyperPure Beads (Roche) and 10 min incubation with mixed beads, and iv) after pre-capture PCR and before ethanol clean-up, beads were eluted in 50 mL of Tris-HCl 10mM pH8.0 for a second incubation with another 50 mL of beads. For the next step, pre-capture libraries were pooled as follows: 1500 ng of four tumor DNA libraries, 1500 ng of eight WBC DNA libraries and 1000ng of one cfDNA library. Capture of the exome was performed with KAPA HyperExome (Roche) following manufacturer's instructions and the same type of post-PCR purification for cfDNA exome samples. The quality of both pre-capture and post-capture libraries was determined with HS D1000 ScreenTape Assay (Agilent) from a twenty-fold dilution of the library. Sequencing was performed on HiSeq 3000 (Illumina) or NovaSeq 6000 (Illumina) with a 150PE and extended i7 read of 17 cycles for UMI reading. The median coverage obtained in the discovery cohort for WBCs was 132X, 194X for the primary tissue and 478X, 504X and 389X for the plasma at baseline, post-operative and relapse stages, respectively.

Validation cohort: Tumor and normal DNA sequencing libraries were generated using xGen UDI-UMI Adapters (Integrated DNA Technologies Inc., IDT) and the Twist Library Preparation Enzymatic Fragmentation Kit 1.0 (TWIST Bioscience). Libraries were prepared as described by the manufacturer. For normal and FrFr DNA, we used 50 ng input and 10 min fragmentation. For FFPE DNA, 200 ng input and 6 min fragmentation were used. All libraries were amplified with seven cycles of PCR. Libraries were quantified using Qubit^{™} dsDNA BR Assay Kit (ThermoFisher), and library size was estimated using TapeStation D1000 (Agilent). Blood samples were collected in K2-EDTA 10 mL tubes (Becton Dickinson) from healthy controls and patients with CRC. Plasma sequencing libraries were prepared using cfDNA from 2 mL of plasma. cfDNA libraries were generated using xGen UDI-UMI Adapters (IDT) and KAPA HyperPrep kit (Roche). Post-ligation clean-up was performed with AMPURE beads in a 1.4x (beads/DNA) ratio to retain short fragments, while post-PCR clean-up was done using a 1.0x ratio. The libraries were amplified with seven cycles of PCR. Libraries were quantified using Qubit^{™} dsDNA BR Assay Kit (ThermoFisher) and library fragment size was estimated using TapeStation D1000 (Agilent). Libraries that did not show the usual bi-modal fragment size distribution13 of cfDNA were excluded before sequencing. Tumor and WBC DNA libraries were captured using the NGS Human Core Exome (TWIST Bioscience, ~33 Mb) according to the manufacturer's protocol. Target-enriched libraries were sequenced using the NovaSeq platform with 2x150 bp paired-end sequencing. The median coverage obtained in the validation cohort for WBCs, primary tissue, plasma at baseline, post-operative and relapse stages was 58X, 95X and 844X, 1022X and 1003X, respectively

### Fastq preprocessing, quality control and read mapping

Raw sample quality control was carried out by FastQC (v0.11.8), whereas Cutadapt (v2.10) was used for the adapter removal and PrinSeq (v0.20.4) to discards reads with mean quality under Q30, in FASTQ preprocessing step. Sequencing reads were mapped to the hg38 human reference genome using BWA (v0.7.17), and BAM postprocessing was performed by PICARD (v2.18.6) and GATK (v4.2.0.0) best practices. Umi-tools (v1.0.1) was used for UMI extraction and deduplication of reads from the same UMI family.

### Variant calling and somatic variant prioritization

Variant calling of primary tumor, plasma and normal samples was performed using combined outputs from Mutect2 (GATK v4.2.0.0) and Lofreq (v2.1.5). For greater confidence, germline variants were also called by HaplotypeCaller (GATK) implemented in Sarek pipeline (v2.7.1). Minimum VAF in primary tumor variants was set to 5% and 0.01% in plasma samples. The final set of variants was yielded by intersecting outputs from the two callers, extraction of variants detected in normal samples and followed by annotation using Variant Effect Predictor (VEP, Ensembl v102). The variants found within a blacklist of redundant mutations were considered sequencing artifacts and were removed. This blacklist was created based on the sequencing of 135 different plasmas. Clonal hematopoiesis of indeterminate potential (CHIP) variants were also removed in plasmas samples to avoid false positives. Somatic pathogenic variants were identified by annotating with COSMIC (v94), OncoKB API (v1) and an in-house pathogenic mutations database. Only high and moderate impact somatic mutations were considered for the mechanistic analysis. A manual review and curation process of the pathogenic mutations detected in each sample was carried out. Variant prioritization analysis, characterized mainly by the match of the pathogenic mutations with OncoKB levels of evidence, was performed to select specific targeted therapies.

### Sequencing quality control

A sequencing quality control criterion was set, requiring sufficient sequencing coverage to ensure that all clonal alterations detected in plasma samples are supported by a minimum of three mutated reads. Tumor purity and CCF were estimated by thePureCN (v.2.0.2) software based on copy number and mutational data. Clonal mutations were defined as those with CCF≥0.9, with the remaining mutations classified as subclonal. Subsequently, the tumor fraction of clonal mutations in all available plasma samples in both cohorts was estimated. The sequencing coverage proved sufficient to meet the quality criterion, ensuring that all clonal mutations in the study's plasma samples with a minimum of three mutated reads were identified

The fraction of clonal and subclonal mutations from the primary tumor detected in the plasma was estimated, obtaining higher sensitivity values when detecting clonal than subclonal mutations in both cohorts. In the discovery cohort, a sensitivity of 29% was observed for clonal mutation identification, which decreased to 11% for subclonal mutations (Wilcoxon paired test; p-value = 0.0005). In the validation cohort, the sensitivity values were 32% and 29% for detection of clonal and subclonal mutations, respectively (Wilcoxon paired test; p-value = 0.0413). The sensitivity to detect subclonal mutations was higher in the validation than the discovery cohort (Wilcoxon test; p-value = 0.0063), given the significantly greater sequencing coverage in these samples.

### TMB and tumor fraction estimation

Tumor Mutational Burden (TMB) was estimated using non-synonymous mutations with an impact on the protein (missense, frame-shift and small indels). Patients with a TMB over 10 mutations/Mb were classified as TMB High. The tumor fraction was estimated as the number of mutated reads (overlapping the mutational compendium of the primary tumor) relative to the total number of reads overlapping the loci of the mutational in the plasma samples.

### Minimal residual disease detection

For increased confidence in variant calling in post-surgery plasma samples, a joint normal sample with a median depth of 3474x was created, consisting of all available WBCs samples. Paired variant calling was performed from this using a heuristic method and a statistical test using the number of aligned reads supporting each allele, based on VarScan2 (v2.4.4) software. A patient was considered positive if at least one somatic mutation was detected through this WES analysis.

To evaluate the clinical feasibility of MRD detection using WES analysis while optimizing cost-effectiveness, we focused on identifying somatic mutations with the highest VAF in the plasma baseline exome of each patient. The objective was to determine whether these mutations remained detectable in postoperative plasma samples. In evaluating sensitivity for MRD detection, we examined various numbers of candidate mutations (ranging from 15 to 20), adhering to the criterion of 1 or 2 detectable mutations necessary to classify a patient as ctDNA positive, consistent with the criteria of existing assays. Results from both the discovery cohort (88% sensitivity with 1 mutation, 67% with two mutations) and the validation cohort (100% with 1 mutation, 86% with two mutations) indicated that sensitivity did not improve with the selection of more than 16 candidate alterations (Fig. 2). Therefore, in a preferred embodiment 16 mutations (TAV16) are selected, aligning with the same number of mutations validated in commercial assays employing a tumor-informed approach for the same purpose.

### Mutational signatures

Known mutation signatures from COSMIC (v3.2 release) were matched with the somatic trinucleotide profile of each plasma and primary tumor sample using SigProfiler (v1.1.3) S.M.A. Islam, Y. Wu, M. Diaz-Gay, et al. Alexandrov, Uncovering novel mutational signatures by de novo extraction with SigProfilerExtractor, BioRxiv (2020) 1-47.

### Copy number variations detection

CNVs in primary tumor samples were called by a combination of CNVkit (0.9.7), VarScan (v2.4.4) and FACETS (v0.15) tools output in a paired tumor-normal mode with a 2000 bp window size. A score-based CNV classification was performed to reduce false positives, considering size and requiring detection by at least two of the tools. CNVs in plasma samples were also called in a paired tumor-normal mode, but using a combination of WisecondorX (Within-SamplE COpy Number aberration DetectOR, v1.2.4) Raman, Lennart, et al. "WisecondorX: improved copy number detection for routine shallow whole-genome sequencing." Nucleic acids research 47.4 (2019): 1605-1614 and CONTRA (v2.0.8) softwares with a 100Kb window size. In plasma samples, a scored-based CNV classification was also used to determine real events.

### Statistical analysis

Normality was checked with the Shapiro-Wilks test. Qualitative variables are presented using frequencies and percentages while quantitative variables are expressed as mean and standard deviation if normality assumption holds true and median and interquartile range (IQR) otherwise. Comparison between continuous variables was carried out using t-Student test if normality criteria was reached; otherwise the Wilcoxon signed-rank test was used. Correlation between quantitative variables was assessed using Spearman's rho statistic. Software used for all analysis was R in its 4.0.1 version (R Core Team, 2021), and the cutoff for statistical significance was set at α = 0.05 in all tests. All tests were two-sided.

### Results

### Patient characteristics

A prospective study, enrolling 320 patients with stage II and III CC between 2015 and 2019 at Hospital Clinico Universitario in Valencia, Spain. Nested within these patients, considered as the discovery cohort, all individuals with recurrence (n=25) who had plasma samples at relapse and tissue samples at baseline (primary tumor) available were selected for WES ctDNA analysis. Patients were predominantly male (15/25, 60%) and had a median age of 74 years. The median time to disease recurrence was 13 months. Relapse sites were diverse, including one (21/25, 84%) or multiple (4/25, 16%) metastatic sites. Most patients (18/25, 72%) received ACT with either capecitabine (12/18, 67%) or CAPOX (6/18, 33%).

In the validation cohort, relapsed CC patients were recruited between 2015 and 2022 at seven Danish hospitals, comparable to the discovery cohort, the median age of the patients was 64 years, and 40% (6/15) were male. The median time to recurrence was 12 months. Among the patients, 67% (10/15) exhibited a solitary relapse site, while 33% (5/15) presented with multiple sites. Most patients (14/15, 93%) received ACT, with treatment regimens including CAPOX (7/14, 50%), FLOX (3/14, 21%), FOLFOX (1/14, 7%), capecitabine (2/4, 14%), or intravenous 5-FU (1/14, 7%).

### Plasma ctDNA analysis reveals intratumor heterogeneity

To demonstrate the capability of plasma ctDNA to provide a comprehensive representation of significant genomic alterations in localized CC, WES was performed on paired plasma and tumor samples from individuals at baseline and relapse. The analysis focused on identifying somatically acquired single nucleotide variants (SNVs), small insertions and deletions (INDELs), and copy number variants (CNVs). All patients in both the discovery and validation cohorts exhibited at least one somatic mutation detected through plasma samples, both at baseline and during relapse. We conducted intra-patient assessment, comparing point somatic mutations and CNVs present in tissue and plasma samples obtained simultaneously. In the discovery cohort, we observed a concordance of 26.6% for all somatic mutations detected in plasma versus tissue samples at baseline (23.2% in the validation cohort, Fig. 4), which dropped to 18.1% when comparing plasma and tissue samples collected at the point of relapse (Fig. 5).

The initial molecular profile revealed a cluster of mutations identified in the tumor tissue but not detected in the paired plasma samples. Inversely, we observed that certain point mutations were exclusive to plasma in some patients; indeed, 33.9% of all somatic mutations were found in plasma only in the discovery cohort at baseline (49% in the validation cohort). This pattern persisted at the time of disease relapse, showing that 22.3% of somatic alterations were exclusively detected in ctDNA. Notably, concordant mutations showed a significantly higher variant allele frequency (VAF) in comparison to plasma-exclusive variants (n=12; t-test; P value = 2.2e-16 in both cohorts).

CNV analysis showed 95.3% concordance between tumor tissue and plasma samples at baseline (n=12) and 90% at relapse (n=17). No significant differences in CNV concordance were observed (n=8, Wilcoxon test, P = 0.1484). Baseline discordance involved 311 genes with loss and 380 genes with gain. Genes with copy number loss in plasma, compared to primary tumors, were enriched in immune signaling pathways, while genes with copy number gain showed enrichment in proliferative signaling pathways.

These results not only highlight the advantage of plasma over tissue in analyzing intratumor heterogeneity (ITH) but also indicate a selective clonal process throughout the course of the disease, emphasizing the importance of plasma-based monitoring and revealing unique genetic signatures that could guide targeted therapeutic interventions for MRD eradication.

### Immediate post-operative ctDNA status association with MRD

To assess whether performing WES on cell-free DNA (cfDNA) from plasma enhances MRD sensitivity detection compared to personalized assays based on a tumor-informed approach or custom panels, we conducted a WES tumor-agnostic (WES-TA) approach on plasma samples collected immediately after curative-intent surgery.

Following plasma sequencing at the postoperative timepoint, at least one somatic mutation was detected in 86.7% (13/15) and 100% (14/14) of patients in the respective cohorts. Additionally, WES data of postoperative plasma from 21 CC patients who had not experienced relapse (from the discovery cohort) revealed only one patient classified as ctDNA positive, yielding a specificity of 95% for this technique.

To assess the clinical applicability of MRD detection utilizing WES analysis within a tumor-informed framework, we focused on the 16 somatic mutations with the highest VAF present in the primary tissue exome of each patient similar to a bespoke commercial assay Reinert T, Henriksen TV, Christensen E, et al. Analysis of Plasma Cell-Free DNA by Ultradeep Sequencing in Patients with Stages I to III Colorectal Cancer. JAMA Oncol 2019;5(8):1124-1131. Henriksen TV, Tarazona N, Frydendahl A, et al. Circulating Tumor DNA in Stage III Colorectal Cancer, beyond Minimal Residual Disease Detection, toward Assessment of Adjuvant Therapy Efficacy and Clinical Behavior of Recurrences. Clin Cancer Res 2022;28(3):507-517

Our analysis to determine whether these mutations were discernible in postoperative plasma samples revealed that at least two of the candidate mutations were detected in plasma in 67% of patients (10/15) in the discovery cohort and 57% of patients (8/14) in the validation cohort (Fig. 6). Conversely, when the selection of the 16 mutations with the highest VAF was based on the analysis of baseline plasma rather than primary tumor specimens (TAV16), a sensitivity of 67% (6/9) in the discovery cohort, and 86% (12/14) in the validation cohort was obtained. However, if we consider that a patient with positive ctDNA is defined by the detection of only one mutation in plasma rather than two, the sensitivity increased to 89% (8/9) in the discovery cohort and 100% (14/14) in the validation cohort. These data suggest that, taking into account that the existing academic and commercial assays require positivity for only a variant in plasma, our WES-TA approach increases sensitivity compared to other current assays while maintaining specificity by detecting a variant.

Additionally, the 16 candidate mutations selected from the WES of primary tumors differed from those identified through the plasma-baseline approach. Comparing both sets, most patients (6/9, 67%) in the discovery cohort had no concordance, resulting in a median 0% concordance rate, while the validation cohort showed 6% concordance (Fig. 7).

Notably, with the tumor-informed approach, 96% and 98% of selected variants were identified as unique to individual patients in the discovery and validation cohorts, respectively. Considering the plasma baseline, in contrast, 86% of the mutations were unique in the discovery cohort and 78% in the validation cohort, supporting a key role for personalized assays in MRD detection.

These findings highlight the potential of utilizing the WES tumor-agnostic approach for monitoring MRD in localized CC, particularly concerning identifying ctDNA positivity with the detection of only one variant in the plasma. Compared to current commercial and academic assays, this method offers greater sensitivity and equal specificity, suggesting possible uses for refining monitoring strategies and promoting precision medicine in this clinical setting.

### Correlation of clonal evolution with tumor progression

To investigate temporal heterogeneity, we conducted WES on cfDNA at both baseline and relapse timepoints. Concordance of somatic variants between plasma samples at baseline and relapse was 61.7% in the discovery cohort and 50.5% in the validation cohort, higher than the concordance observed between primary tumor and ctDNA at relapse (Fig. 8, 27.7%; n = 12; Wilcoxon test; P = 0.0015), as well as primary tumor and ctDNA post-surgery (Fig. 9), reflecting both intratumor heterogeneity and plasma's better ability to capture clonal evolution. Furthermore, concordance between primary tumor and paired metastatic tissue was higher in patients with a single metastatic lesion compared to those with multiple lesions (multiple, n = 10, 29.75%; single, n = 7, 80.87%; Wilcoxon test, P = 0.0068). A similar observation was made when comparing baseline plasma to metastatic tissue (multiple, n = 5, 25.55%; single, n = 3, 80.87%; Wilcoxon-test, P value = 0.0357) and when comparing recurrence plasma to metastatic tissue (multiple, n = 10, 11.96%; single, n = 7, 32.66%; Wilcoxon-test, P value = 0.0054). On the other hand, the concordance between baseline and relapse plasma is not significantly influenced by the presence of single or multiple metastases (multiple, n = 6, 57.56%; single, n = 6, 62.43%; Wilcoxon test, p-value = 0.6991), further highlighting the tumor's limitation in comprehensively capturing ITH.

Gaining a comprehensive understanding of tumor evolutionary dynamics in CC patients; considering all detected mutations at each timepoint, allowed us to discern alterations that diminish during the evolutionary process, those that endure over time, and those that surface at the point of relapse (Fig. 10). We detected acquired variants in ctDNA at the time of relapse, constituting 23% and 26.5% of somatic mutations in the discovery and validation cohorts, respectively.

Notable individual heterogeneity was observed in the tempo of tumor evolution. Some patients showed gradual changes, with mutations appearing at relapse absent in baseline plasma samples, regardless of chemotherapy. Transcriptomic deconvolution in primary tissues from the discovery cohort revealed that molecular similarity between baseline and relapse plasma correlated with activated B cells (Spearman; n = 7; P = 0.0366), confirmed by immunohistochemistry (IHC) in both cohorts (discovery: n = 7, P = 0.0215; validation: n = 14, P = 0.0460). Elevated infiltration at diagnosis was linked to reduced baseline-relapse concordance, indicating more rapid tumor evolution, while lower infiltration and higher similarity suggested slower evolution.

Our analysis showed that somatic mutations acquired during relapse in both the discovery and validation cohorts were significantly associated with activation of the epithelial-mesenchymal transition (EMT) pathway. This was confirmed in the genomic profiles of relapsing tissue samples, where the EMT pathway was overrepresented in mutations acquired during relapse in metastatic lesions. Transcriptomic profiling revealed that wild-type patients for EMT genes did not undergo the transition, while mutated patients shifted from an epithelial to a mesenchymal profile due to EMT pathway activation. These patients carried high-impact mutations in FLNA, ITGB3, LAMC1, SLIT3, and TGFBR3, known to activate the EMT pathway.

Interestingly, we observed a significant enrichment in loss of heterozygosity in genes related to myogenesis (P value = 6.759e-05; MYH1, MYH2, MYH3, MYH4, MYH8, and CHRNB1) at baseline, not identified at relapse.

Our findings suggest that tumoral evolution is more accurately captured through ctDNA analysis, and also identify two evolution patterns associated with initial B cell infiltration into the primary tumor, which may contribute to immune evasion by tumor cells and subsequent cellular migration to other organs.

### ctDNA parallel evolution analysis reveals tumor progression

To investigate the mechanisms underlying localized CC progression, we conducted an analysis of acquired somatic mutations at the time of relapse and their associated functions. Specifically, the number of mutations per gene present in plasma samples were examined at both baseline and relapse to investigate the parallel evolution of the tumor.

In both the discovery and validation cohorts, no significant differences in tumor mutational burden (TMB) at relapse compared to baseline were observed in either tissue or plasma. In this context, the dN/dS ratio is a valuable metric for assessing the strength and mode of natural selection on protein-coding genes Martincorena I, Raine KM, Gerstung M, et al. Universal Patterns of Selection in Cancer and Somatic Tissues. Cell 2017;171(5):1029-1041.e21.

We next examined the potential correlation between dN/dS and TMB, both at the time of diagnosis and relapse. Our analysis revealed that at diagnosis no significant correlation could be established between these parameters in either tissue or plasma. In the discovery cohort, however, a noteworthy correlation between these variables emerged at the point of relapse, in both plasma and tissue samples (Spearman correlation; plasma: n = 12, P value = 0.0228, tissue, n = 25, P value = 0.0199). This significant correlation at the time of recurrence was also observed in the validation cohort when comparing those parameters in plasma (Spearman correlation; n = 15, P value = 0.0321).

The correlation between TMB and dN/dS ratio during relapse suggests that tumors accumulate numerous pathogenic alterations, driven by positive evolutionary selection, leading to functions crucial for tumor progression. Our study focused on genes with significantly increased mutations at relapse compared to diagnosis, excluding MSI patients due to high TMB. In the discovery cohort, 115 genes met this criterion, with GOLGA6, HLA, and PABP gene families notable in both cohorts. KEGG functional enrichment analysis highlighted the involvement of hypermutated genes in immune evasion pathways, including antigen presentation and processing, which was consistently observed in the validation cohort.

### Experiment 2: Use of the method of the invention with particular subjects

### Case 1: ctDNA negative patient

A 64-year-old male patient with no relevant medical history was diagnosed with right colon adenocarcinoma and underwent curative-intent surgery in October 2021. Pathological staging confirmed pT4pN2M0 (Stage III high-risk). The patient's carcinoembryonic antigen (CEA) levels remained within the normal range both pre- and post-surgery, providing no additional prognostic information. Based on current clinical guidelines, the patient was recommended to undergo adjuvant chemotherapy with CAPOX for six months. However, after four cycles of oxaliplatin, the patient developed grade 3 toxicity, leading to early treatment discontinuation. Despite receiving a reduced chemotherapy regimen, the patient remained disease-free for 36 months, raising the question of whether adjuvant chemotherapy had been necessary in this case.

Currently, all high-risk Stage III colorectal cancer patients receive adjuvant chemotherapy, despite the fact that a subset of them may not harbor MRD and derive no benefit from additional treatment. The presence of ctDNA after surgery has been strongly correlated with recurrence risk, making it a valuable biomarker for guiding post-surgical therapeutic decisions. The method of the invention is a tumor-agnostic MRD detection method that enables highly sensitive ctDNA analysis without requiring prior tumor sequencing, allowing real-time assessment of disease status. By identifying patients who are truly MRD-negative, it has the potential to prevent unnecessary chemotherapy administration, reducing treatment-related toxicity and improving patient quality of life.

Prior to surgery, a baseline blood sample was collected, consisting of plasma and WBCs. Plasma was used for ctDNA analysis, while WBCs served as a germline control to eliminate clonal hematopoiesis-derived variants. The plasma sample underwent WES, allowing for the identification of 16 high-allelic frequency somatic mutations that were tumor-derived. These mutations were selected as patient-specific markers for post-surgical MRD assessment.

At 4 weeks post-surgery, a single plasma sample was collected for MRD detection. This sample was processed using amplicon-based ultra-deep sequencing, targeting the previously identified 16 mutations with high sensitivity and specificity.

As a result of the bioinformatics analysis, the post-surgical ctDNA analysis revealed no detectable tumor-derived mutations, indicating a negative MRD status. Given the well-established correlation between MRD negativity and long-term recurrence-free survival, this result suggested that the patient had no residual disease after surgery and was unlikely to benefit from adjuvant chemotherapy. Had this information been available at the time of treatment planning, chemotherapy could have been safely omitted, avoiding unnecessary toxicity and improving the patient's quality of life.

This case highlights the clinical utility of the method of the invention in guiding adjuvant therapy decisions for Stage III colorectal cancer patients. Under current clinical practice, all high-risk patients receive chemotherapy, despite a significant proportion of them being MRD-negative and unlikely to relapse. By integrating TAV16-based MRD detection into standard post-surgical workflows, oncologists could identify patients who do not require additional treatment, reducing overtreatment, minimizing toxicity, and preserving patient quality of life while maintaining optimal oncological outcomes.

### Case 2: ctDNA positive patient

A 58-year-old female patient with no significant medical history was diagnosed with sigmoid colon adenocarcinoma and underwent curative-intent surgery in June 2022. Pathological staging confirmed pT3pN1M0 (Stage IIIA). The patient's CEA levels were within the normal range both pre- and post-surgery, offering no additional prognostic insight. Based on current guidelines, adjuvant chemotherapy with CAPOX for six months was recommended. However, recent studies suggest that not all Stage III patients require chemotherapy, making MRD detection via ctDNA a key tool for personalized treatment decisions.

The persistence of ctDNA after curative-intent surgery has been shown to strongly correlate with recurrence risk, providing an opportunity for early therapeutic intervention in patients with residual disease. TAV16 is a tumor-agnostic MRD detection method that enables highly sensitive ctDNA analysis without the need for prior tumor sequencing. By identifying patients with detectable ctDNA post-surgery, TAV16 facilitates stratification into clinical trials and personalized treatment approaches aimed at preventing relapse.

Prior to surgery, a baseline blood sample was collected, consisting of plasma and WBC. Plasma was used for ctDNA analysis, while WBCs served as a germline control to eliminate clonal hematopoiesis-derived variants. The plasma sample underwent WES, allowing for the identification of 16 high-allelic frequency somatic mutations that were tumor-derived. These mutations were selected as patient-specific markers for post-surgical MRD assessment.

At 4 weeks post-surgery, a single plasma sample was collected for MRD detection. This sample was analyzed using amplicon-based ultra-deep sequencing, focusing on the previously identified 16 mutations with high sensitivity and specificity.

As a result of the bioinformatics analysis, the post-surgical ctDNA analysis revealed one detectable tumor-derived mutation, indicating a positive MRD status. Given the strong correlation between ctDNA positivity and disease recurrence, the patient was considered to be at high risk of relapse despite having undergone complete surgical resection. As a result, the patient was enrolled in a clinical trial investigating an intensified adjuvant therapy regimen for MRD-positive colorectal cancer patients.

The patient received a combination of CAPOX and an investigational targeted agent designed to enhance eradication of micrometastatic disease. Serial plasma samples were collected every three months to monitor ctDNA dynamics over time. By the six-month follow-up, a decline in ctDNA levels was observed, and by nine months post-surgery, ctDNA was no longer detectable, indicating a negative MRD status. The patient completed the full treatment regimen and, after 24 months of follow-up, showed no radiological or molecular evidence of disease recurrence, achieving a complete response.

This case highlights the clinical utility of TAV16 in identifying MRD-positive patients who may benefit from early therapeutic intervention. Under conventional clinical practice, this patient would have received standard CAPOX therapy without knowledge of their high risk of relapse. The method of the invention allows to identify high-risk patients, offer enrollment in clinical trials, and monitor ctDNA clearance as a surrogate marker of treatment response, ultimately improving long-term patient outcomes.

## Claims

1. A method for detecting minimal residual disease in a subject suffering or that has suffered cancer, comprising the following steps:
(a) assaying cell free deoxynucleic acids cfDNA from a plasma sample obtained from said subject at a first time point; the first-time point being pre-surgery,
(b) detecting altered variants from said cfDNA from a plasma sample; by using whole exome sequencing; using genomic DNA from white blood cells, from the same subject as germline control,
(c) computer processing the differentially expressed variants to determine the most relevant differentially expressed variants, and
(d) generating an amplicon panel design that comprises sequences of at the at least 13 most relevant altered variants of step c);
assaying
cfDNA from a plasma sample from the same subject obtained at a second time point, which is after surgery; and
cfDNA from plasma samples from a pool of healthy subjects as control;
with said amplicon panel, to call the presence of least one of said most relevant altered variants,
wherein the calling of at least one of said most relevant altered variants in the plasma sample obtained at a second time point indicates the presence of minimal residual disease in said subject.

2. The method according to the preceding claim, wherein steps are performed in order a) to d).

3. The method according to any of the preceding claims, wherein pre-surgery means at least 2 days before the surgery.

4. The method according to any of the preceding claims, wherein the variants are one or more of the following copy number alterations, copy number losses, single nucleotide variants, insertions or deletions, and/ or rearrangements.

5. The method according to any of the preceding claims, wherein step c) comprises the steps, carried out by a computer of: Quality control; Adapter trimming and quality filtering; Read alignment; Binary alignment; UMI processing; Variant calling; Germline variant detection; Variant filtering; Variant annotation and selecting of the the most relevant altered variants

6. The method according to any of the preceding claims, wherein the cfDNA whole exome sequencing coverage is at least 50X

7. The method according to any of the preceding claims, wherein the most relevant differentially expressed variants are the 13 variants with the highest variant allele frequency, classified as "high" or "moderate", with a population frequency of less than 5%.

8. The method according to any of the preceding claims, wherein the second time point is at least 1 week after the first time-point, preferably 3 to 4 weeks after surgery.

9. The method according to any of the preceding claims, wherein step d) comprises the steps, carried out by a computer of: Quality control; Adapter trimming and quality filtering; Read alignment; BAM Post-Processing; UMI processing and error correction; Variant calling with background error correction; and detection of at least one of the most relevant altered variants identified in step c)

10. The method according to any of the preceding claims, wherein the sequencing coverage at step d) is at least 20,000× depth

11. The method according to any of the preceding claims, wherein the cfDNA whole exome sequencing coverage is at least 350X and the sequencing coverage at step d) is at least 100,000× depth.

12. The method according to any of the preceding claims, wherein the cancer is selected from the group consisting of: genitourinary cancer, breast cancer, lung cancer, prostate cancer, colorectal cancer, melanoma, bladder cancer, non-Hodgkin lymphoma, kidney cancer, endometrial cancer, leukemia, pancreatic cancer, thyroid cancer, and liver cancer, and any combination thereof, preferably colorectal cancer

13. The method according to any of the preceding claims, wherein the detection of at least one of the most relevant differentially expressed variants, in the plasma sample of the second time point indicates the presence of MRD in the subject.

14. The method according to any of the preceding claims, wherein one or more plasma samples of step d) are obtained at different time points after the second time point.

15. A method for detecting molecular relapse in a subject suffering or that has suffered cancer, comprising the following steps:
(a) assaying cell free deoxynucleic acids cfDNA from a plasma sample obtained from said subject at a first time point; the first-time point being pre-surgery,
(b) detecting altered variants from said cfDNA from a plasma sample; by using whole exome sequencing; using genomic DNA from white blood cells, from the same subject as germline control,
(c) computer processing the differentially expressed variants to determine the most relevant differentially expressed variants, and
(d) generating an amplicon panel design that comprises sequences of at the at least 13 most relevant altered variants of step c);
assaying
cfDNA from a plasma sample from the same subject obtained at two or more time points after surgery; and
cfDNA from plasma samples from a pool of healthy subjects, as control; with said amplicon panel, to call the presence of least one of said most
relevant altered variants,
wherein the calling of said at least one of said most relevant altered variants in at least one plasma sample obtained after surgery indicates molecular relapse in said subject.
